# DEMANDE DE BREVET EUROPEEN

(11) **EP 4 056 165 A2**
(43) Date de publication de la demande: **14.09.2022**
(21) Numéro de dépôt: 22020058.8
(22) Date de dépôt: 17.02.2022
(51) Int. Cl.: A61K 8/11, A61K 8/9783, A61Q 1/04

(54) **MATIÈRE PREMIÈRE COLORANTE ISSUE DE PLANTES SELON UN PROCÉDÉ METTANT EN OEUVRE UNE EXTRACTION PARTICULIÈRE ET UN ENROBAGE PARTICULIER**

(30) Priorité: 19.02.2021 FR 2101619
(71) Demandeur: Le Rouge Français, 92140 Clamart (FR)
(72) Inventeur: Carpentier, Elodie, 92140 Clamart (FR)

(57) **Abrégé**

Matière première colorante constituée d'un extrait colorant issu de la totalité ou d'une ou plusieurs parties d'une ou plusieurs plantes à partir d'un procédé comprenant une phase d' extraction avec au moins une extraction avec action d'un ou plusieurs agents exogènes choisis parmi les solvants sous pression, les enzymes, les ondes radioélectriques de fréquences allant de 16 Khz à 300 GHz et un enrobage de l'extrait obtenu par un ou plusieurs agents issus de plantes, notamment choisis parmi les polysaccharides et les polypeptides dont les protéines. Composition comprenant cette matière colorante, utilisée en particulier dans le domaine cosmétique. Procédé de maquillage, notamment de la peau ou des lèvres ou des cils ou des ongles, mettant en œuvre une telle composition.

## Description

Dans de nombreux domaines pour apporter de la couleur à un substrat il est connu d'utiliser des matières premières colorantes qui sont soit des colorants solubles soit des pigments insolubles. Les espèces colorantes peuvent être d'origine naturelle ou synthétique. Beaucoup de colorants ou de pigments colorés d'origine synthétique posent des problèmes d'innocuité, notamment au niveau des risques de génotoxicité ou de potentiel sensibilisant. Par ailleurs ces colorants ou pigments synthétiques ne sont pas issus de sources renouvelables et leur empreinte carbone n'est pas favorable.

Dans les matières colorantes d'origine naturelle on utilise parfois des espèces d'origine minérale mais d'une part la palette des teintes obtensibles est limitée et d'autre part on peut se retrouver face à des problèmes de traces de métaux lourds.

Il est donc logique de se tourner vers les espèces colorantes issues du monde vivant et tout particulièrement pour des questions éthiques vers les espèces colorantes issues du monde végétal.

Les problèmes auxquels on se trouve alors confronté se situent à plusieurs niveaux.

Le premier niveau est relatif aux concentrations relativement faibles en molécules colorantes actives présentes dans les végétaux. Il s'avère que souvent les extractions de ces molécules colorantes à partir des végétaux concernés sont réalisées avec de l'eau à pression atmosphérique éventuellement en chauffant en évitant les températures trop élevées pour éviter les risques de dégradation. Les rendements d'extraction sont alors assez faibles et les produits obtenus peu concentrés.

Le second niveau se situe au niveau des propriétés intrinsèques des espèces colorantes d'origine végétale. Beaucoup d'entre elles sont relativement fragiles notamment sur le plan de la stabilité face à l'action de composés chimiques ou à des variations de pH, ou sur le plan de la résistance à la lumière naturelle. Enfin certaines d'entre elles se dispersent difficilement dans les milieux de formulation.

Le troisième niveau se situe au niveau de propriétés connexes de l'extrait. En particulier très souvent l'extraction n'est pas univoque et on retrouve dans l'extrait final des composés gênants comme ceux conférant à l'extrait une mauvaise odeur.

Tout ce qui vient d'être dit est vrai pour de nombreux domaines d'utilisation, par exemple dans le domaine alimentaire. C'est aussi particulièrement vrai pour le domaine de la cosmétique où existe un véritable besoin de modifier la couleur des différents substrats présents sur le corps humain.

Il a été proposé dans le document MY161184A de l'Université de Technologie Mara des nanocolorants avec des pigments obtenus à partir d'anthocyanes de fleurs et en-capsulés dans des microparticules de chitosane ou d'alginate. Outre le fait qu'ils s'agit de pigments végétaux particuliers et que l'extraction est réalisée par utilisation d'un solvant choisi parmi l'eau, les solvants organiques miscibles à l'eau ou leurs mélanges et ceci à pression atmosphérique, ce qui limite les rendements d'extraction, on notera que le chitosane est d'origine animale et que ses sources habituelles sont des crustacés ce qui peut induire des risques de sensibilisation chez l'être humain. Les alginates sont quant à eux issus des algues brunes qui appartiennent au règne des Chromistes, un des six règnes d'eucaryotes. Ces alginates ont comme principaux défauts une sensibilité aux ions calcium (gélification hétérogène), des problèmes potentiels de coloration parasite et/ ou d'odeur résiduelle désagréable dans leur mise en oeuvre.

Il existe donc toujours un besoin de mettre à disposition de nouvelles matières premières colorantes permettant de surmonter les problèmes évoqués ci-dessus.

A cet effet, la demanderesse préconise une matière première colorante constituée d'un extrait colorant issu de la totalité ou d'une ou plusieurs parties d'une ou plusieurs plantes à partir d'un procédé comprenant les étapes suivantes:
- (a) une phase d'extraction comprenant au moins une extraction (aᵢ) avec action d'un ou plusieurs agents exogènes choisis parmi les solvants sous pression, les enzymes, les ondes radioélectriques de fréquences allant de 16 Khz à 300 GHz.
- (b) un enrobage de l'extrait issu de la phase d'extraction (a) avec un ou plusieurs agents issus d'une ou plusieurs plantes.

Par plante on entend au sens de la présente invention un organisme appartenant au règne Plante (ou Plantae), un des six règnes du domaine (ou empire ou super-règne) des eucaryotes dans la classification de RUGGIERO et All (2015), les cinq autres étant les Protozoaires (Protozoa), les Chromistes (Chromista), les Champignons ou mycètes (Fungi) et les Animaux (Animalia).

Outre son origine naturelle, la matière première colorante obtenue possède une efficacité accrue ainsi qu'un maintien de cette efficacité dans le temps ou face à la lumière tout en évitant des problèmes comme les problèmes éventuels d'odeur ou de coloration intempestives liées aux plantes soumises à l'extraction ou aux éventuels résidus de solvants, les problèmes d'innocuité potentielle des extraits obtenus dans la phase (a), et les problèmes de mise en oeuvre des matières premières colorantes dans certains domaines tels que la cosmétique.

Par extrait colorant de plante on entend au sens de la présente invention un extrait contenant un ou plusieurs colorants issus d'une plante ou d'un mélange de plantes ou de sécrétions d'une ou plusieurs plantes.

Les extraits colorants de plantes peuvent provenir de tous types de végétaux appartenant au règne Plante des eucaryotes défini ci-dessus. Ils peuvent notamment provenir de phanérogames. Ils peuvent en particulier provenir d'arbres, d'arbustes, de plantes vertes, de plantes à fleurs, de cactées, de graminées. Ils peuvent provenir de toutes les parties de la plante et en particulier de la plante entière ou des racines, des tiges ou des troncs et notamment des écorces, des feuilles, des fleurs, des graines et des fruits. Ils peuvent aussi être issus de sécrétions ou exsudats de plantes produits par les dites plantes, en particulier en réaction ou en prévention à une agression. A titre d'extraits colorants de plantes on peut notamment citer de manière non limitative ceux issus de:
Garance (exemple d'espèce Rubia Tinctorum)
Tomate (exemple d'espèce Solanum Lycopersicum)
Quebracho(exemple d'espèce Schinopsis quebracho-colorado)
Pernambouc (exemple d'espèce Caesalpinia Echinata)
Châtaignier (exemple d'espèce Castanea Sativa)
Curcuma (exemple d'espèce Curcuma Tumeric)
Sorgho (exermple d'espèce Sorghum Bicolor)
Cachou (exemple d'espèce Acacia Catechu)
Rocouyer (exemple d'espèce Bixa Orellana)
Sappan (exemple d'espèce Caesalpinia Sappan)
Genipa (exemple d'espèce Genipa Americana)
Indigotier (exemple d'espèce Indigofera Tinctoria)
Chou rouge (exemple d'espèce Brassica Oleracea)
Réséda (exemple d'espèce Reseda Luteola)
Sumac (exemple d'espèce Rhus Semialata)
Radis rouge (exemple d'espèse Raphanus Sativus)
De préférence les extraits colorants de plantes sont choisis parmi ceux issus de la garance, de la tomate, du sorgho, du châtaignier, du radis rouge, du sumac.

La phase d'extraction (a) comprend au moins une extraction (aᵢ) en présence d'un ou plusieurs agents exogènes choisi parmi les solvants sous pression, les enzymes, les ondes radioélectriques de fréquences allant de 16 Khz à 300 GHz.

Par agent exogène on entend au sens de la présente invention un agent extérieur intervenant en complément de la plante ou partie de plante utilisée.

L'agent exogène peut être un rayonnement radioélectrique de fréquences allant de 16 KHz à 300 GHz. De préférence le rayonnement radioélectrique se situe dans le domaine de 16 Khz à 10 MGHz (domaine des ultrasons) ou dans le domaine de 500 MHz à 300 GHz (domaine des micro-ondes). Dans l'étape (aᵢ) on peut éventuellement utiliser deux types de rayonnements radioélectriques appartenant à la gamme principale de fréquences indiquée et notamment les deux domaines précités.

L'agent exogène peut être un composé choisi parmi les solvants sous pression.

Par sous pression on entend que la pression d'utilisation du solvant est supérieure à la pression atmosphérique (1,013×10⁵ Pa).

Par solvant on entend une substance qui, dans ses conditions d'utilisation, a le pouvoir de dissoudre d'autres substances sans les modifier chimiquement et sans elle-même se modifier.

De préférence les solvants de l'invention possèdent de 0 à 12 atomes de carbone, mieux de 0 à 5 atomes de carbone.

Les solvants ou mélanges de solvants utilisés peuvent être des liquides à température ambiante (25°C) et à pression atmosphérique (1,013×10⁵ Pa). Ils peuvent être aussi des gaz à température ambiante (25°C) et à pression atmosphérique (1,013×10⁵ Pa) qu'on utilise alors sous pression sous forme liquide ou pseudo liquide.

L'extraction par liquide pressurisé (pressurized liquid extraction, PLE), aussi appelée extraction par solvant accélérée (accelerated solvent extraction, ASE) ou aussi extraction par fluide pressurisé (pressurized fluid extraction, PFE) est une technique d'extraction solide-liquide. Elle a l'avantage dans l'invention de permettre d'effectuer des extractions beaucoup plus rapides et utilisant moins de solvants avec une meilleure solubilisation des actifs colorants.

Les solvants liquides à température ambiante (25°C) et à pression atmosphérique (1,013×10⁵ Pa) utilisés sous pression peuvent être choisis parmi l'hexane, l'acétate d'éthyle, l'acétone, l'eau, l'éthanol et leurs mélanges et de préférence parmi l'éthanol et les mélanges éthanol/eau.

Les solvants gazeux à température ambiante (25°C) et à pression atmosphérique (1,013×10⁵ Pa) utilisés sous pression peuvent être choisis parmi le diméthyl éther, les alcanes en C3-C5 tels que le n butane, les alcanes ou alcènes fluorés tels que le 1,1 difluoroéthane, le 1,1,1,2 tétrafluoroéthane, le 1,3,3,3 tétrafluoro prop-1-ène.

On peut aussi utiliser des fluides supercritiques.

Par fluide supercritique on entend un composé maintenu au delà de son point critique, c'est à dire chauffé au delà de sa température critique lorsqu'il est comprimé au dessus de sa pression critique. Le domaine d'existence d'un fluide supercritique est défini par le diagramme de phases pression-température du composé correspondant, le point critique étant le point où s'arrête la courbe d'équilibre liquide-gaz. Les fluides supercritiques ont un comportement pseudo-liquide.

Par pseudo-liquide on entend l'état d'une substance présentant une densité intermédiaire entre celle de la phase liquide et celle de la phase gazeuse. Ces fluides supercritiques allient donc densité proche d'un liquide et diffusivité d'un gaz.

De préférence le fluide supercritique utilisé est le dioxyde de carbone supercritique pour lequel le point critique est défini par la température critique de 31°C et la pression critique de 73,8 bars (soit 7,38 MPa , 1 MPa étant équivalent à 10⁶ Pa et 1 bar à 10⁵ Pa).

Les solvants sous pression de l'invention peuvent être utilisés à une pression allant de préférence de 1 MPa à 200 MPa , mieux de 3 Mpa à 150 Mpa, encore mieux de 10 Mpa à 150 Mpa et encore plus préférentiellement de 10 MPa à 100 Mpa.

L'extraction (aᵢ) peut se faire en présence d'une ou plusieurs enzymes. Ces enzymes sont de préférence choisies parmi les hydrolases et les lyases.

A titre de lyases utilisables on peut citer les décarboxylases, les aldolases et les déshydratases.

On préférera utiliser les hydrolases et en particulier les estérases, les peptidases, les glycosidases et les phosphatases. On utilisera par exemple les cellulases, les bêta-glucosidases, les hémicellulases, les xylanases, les glucanases, les bêta-glucanases, les pectinases, les amylases, les alpha-amylases, les phospholipases, les bêta-mannanases,les galactanases, les arabinanases, les laminarinases, les galacturonases, les polygalacturonases, les phytases, les exocellulases, les phénylestérases et les protéases.

L'extraction (aᵢ) mettant en œuvre au moins un agent exogène tel que décrit ci-dessus peut s'effectuer à température peu élevée sans chauffage, notamment de 4°C à 40°C, mieux de 20°C à 40°C ou en chauffant. La température de chauffage va de préférence de 41°C à 200°C, mieux de 45°C à 160°C, encore mieux de 45°C à 100°C.

On peut bien entendu combiner ces technologies et notamment:
- enzymes et ondes radioélectriques
- solvants ou mélanges de solvants sous pression, et ondes radioélectriques ,les technologies étant telles que définies ci-dessus.
L'extraction (aᵢ) peut aussi se faire en présence d'autres agents exogènes additionnels différents de ceux mentionnés ci dessus. On citera tout particulièrement les agents chimiques d'origine naturelle ou non permettant une rupture de certaines liaisons chimiques tels que les agents réducteurs ou oxydants ou les agents d'hydrolyse acides ou basiques. Dans certains cas on pourra utiliser à titre d'agents exogènes additionnels les solvants à pression atmosphérique.
Par ailleurs le procédé de préparation de la matière colorante selon l'invention peut comporter dans la phase d'extraction (a) plusieurs étapes d'extraction séparées dont au moins une conforme à l'extraction (aᵢ) telle que décrite ci-dessus. De préférence lorqu'il y a plusieurs étapes d'extraction, l'étape d'extraction (aᵢ) caractéristique de l'invention précède la ou les suivantes.

L'étape (b) du procédé de préparation de la matière première colorante de l'invention est un enrobage de l'extrait issu de la phase d'extraction avec un ou plusieurs agents issus d'une ou plusieurs plantes.

Par enrobage au sens de la présente invention on entend un procédé permettant d'isoler l'extrait du milieu environnant. Cet enrobage peut résulter d'un dépôt d'un ou plusieurs matériaux solides ou liquides sur l'extrait ou de l'inclusion de l'extrait dans une particule formée par un ou plusieurs matériaux, les dits matériaux étant issus de tous types de végétaux appartenant au règne Plante des eucaryotes tel que défini ci-dessus. Les particules évoquées sont de préférences des microparticules. Si elles sont creuses on parlera de microencapsulation. Si elles sont pleines avec des espaces d'inclusion on parlera de microsphères.

Le dépôt simple peut se faire par des techniques de pulvérisation, d'attraction, de friction ou de cristallisation.

Les principales technique d'encapsulation utilisables sont l'atomisation (spray drying), l'extrusion, la coacervation, la formation de liposomes, la gélification ionique, la lyo-phylisation, le lit-fluidisé, l'émulsification via des émulsions multiples.

Les matériaux ou agents utilisables dans l'enrobage de l'étape (b) peuvent être des corps gras, des sucres, des polysaccharides, des acides aminés, des peptides voire des associations de cellules issues par exemples du fractionnement de végétaux. L'enrobage est de préférence réalisée avec:
- des polysaccharides
- des polypeptides dont les protéines

Les polysaccharides utilisés pour l'enrobage de l'étape (b) sont de préférence des dextrines dont les matodextrines et les cyclodextrines, des dextranes, des celluloses, des amidons, des pectines.

Les polypeptides utilisés pour l'enrobage de l'étape (b) peuvent être notamment des protéines telles que des prolamines, des albumines, des globulines, des gluténines et leurs mélanges.

Les polypeptides d'enrobage peuvent être des protéines issues de plantes choisies parmi les céréales, les légumineuses, les oléagineuses et de préférence parmi le blé, le maïs, l'orge, l'avoine, le soja, le coton, le riz, le pois chiche, le petit pois, le seigle, le froment, le millet, la fève, le lupin, la lentille, l'épautre, le sésame, le sarrazin, le fénugrec, le tournesol, le lin.

Avantageusement on utlisera à titre d'agent d'enrobage au moins une dextrine ou un dextrane.

Le procédé d'obtention des matières colorantes de l'invention peut comprendre en plus d'autres étapes que les étapes d'extraction et d'enrobage précédemment décrites.

Parmi ces étapes complémentaires on peut citer celles mettant en œuvre une ou plusieurs des opérations suivantes : broyage, filtration, évaporation ou concentration, tamisage, séchage, lyophilisation.

Certaines de ces autres étapes peuvent prendre place avant la phase (a), après la phase (a), après l'étape (b), ou entre deux étapes d'extraction de la phase (a).

• Les matières premières colorantes de l'invention sont mises en œuvre dans des compositions. Ces compositions contiennent une ou plusieurs matières premières colorantes selon l'invention.

La concentration en matières premières colorantes de l'invention dans ces compositions peut varier de 0,001% à 60% en poids, plus préférentiellement de 0,1% à 50% en poids, mieux de 0,5% à 40% en poids, encore mieux de 1% à 35% en poids par rapport au poids total de la composition.

Les matières colorants de l'invention peuvent être complètement solubilisées ou de préférence dispersées dans la composition les contenant. Par dispersée on entend que la matière colorante est insolubilisée ou pas complètement solubilisée dans la composition à température ambiante et à pression atmosphérique.

Il est possible d'ajouter aux matières colorantes de l'invention dans les compositions selon l'invention d'autres matières colorantes soit solubles dans l'eau, soit insolubles dans l'eau à température ambiante et à pression atmosphérique. Ces matières colorantes additionnelles peuvent être des colorants naturels dont les colorants issus de plantes mais obtenus par un procédé différent de celui de l'invention. Ces matières colorantes additionnelles peuvent être aussi des pigments ou des colorants solubles organiques de synthèse ou des pigments minéraux.

De préférence, les matières colorantes des compositions selon l'invention sont toutes d'origine végétale.

Les compositions de l'invention peuvent être anhydres ou aqueuses.

Par anhydre on entend au sens de la présente invention une composition contenant à température ambiante et à pression atmosphérique moins de 5% d'eau en poids, encore mieux moins de 1% d'eau en poids par rapport au poids total de la composition et préférentiellement ne contenant pas d'eau, en particulier d'eau ajoutée.

Par aqueuse on entend au sens de la présente invention une composition contenant à température ambiante et à pression atmosphérique de 5% à 99,01% d'eau en poids par rapport au poids total de la composition.

De préférence si elle est aqueuse, la composition comprend à température ambiante et à pression atmosphérique de 25% à 98% d'eau en poids, mieux de 40% à 95% d'eau en poids par rapport au poids total de la composition.

Les compositions selon l'invention peuvent se présenter sous forme de poudres, de solides compacts tels que des sticks, de liquides, de gels, d'émulsions simples ou multiples, de mousses aérosols.

Les compositions selon l'invention contiennent de préférence un ou plusieurs corps gras.

Le ou les corps gras sont choisis parmi les huiles ou les cires.

Les compositions de l'invention peuvent contenir une ou plusieurs huiles.

Par huile on entend au sens de la présente invention un composé non miscible à l'eau liquide à température ambiante et à pression atmosphérique.

Les huiles peuvent être polaires ou apolaires, volatiles ou non volatiles (une huile est dite non volatile quand sa pression de vapeur est inférieure à 0,02 mm de mercure à pression atmosphérique et à température ambiante).

Les huiles peuvent être hydrocarbonées, d'origine végétale, minérale ou synthétique. Elles peuvent être aussi siliconées ou fluorées.

Les huiles siliconées peuvent être des polydiméthylsiloxanes linéaires ou ramifiés ou cycliques porteurs éventuellement de groupes aryle et en particulier phényle.

Les huiles hydrocarbonées apolaires sont choisies de préférence parmi l'huile de paraffine, les isoparaffines (isododécane, isodécanes), les alcanes linéaires (n-dodécane), le squalane, l'eicosane, les polybutylènes ou les polyisobutylènes hydrogénés ou non, les polydècènes hydrogénés ou non.

Les huiles polaires sont de préférence hydrocarbonées. Elles peuvent être choisies parmi les alcools gras ramifiés ou insaturés comme l'alcool oléique et l'octyldodécanol. Elles sont de préférence choisies parmi les triglycérides d'origine végétale ou synthétique et les esters d'acides gras autres que les triglycérides. Parmi les huiles d'origine végétale on peut notamment citer l'huile d'amande douce,

l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyaux d'abricot, l'huile d'olive, l'huile d'onagre, l'huile de palme, l'huile de passiflore, l'huile de pépins de raisin, l'huile de ricin, l'huile de rosier, l'huile de seigle, l'huile de sésame, l'huile de son de riz, l'huile de soja et l'huile de tournesol.

De préférence l'huile ou les huiles est ou sont choisies parmi les huiles végétales.

Les compositions selon l'invention peuvent contenir une ou plusieurs cires.

Par cire on entend au sens de la présente invention un composé lipophile solide à température ambiante et à pression atmosphérique, à changement d'état solide/liquide réversible, ayant un point de fusion supérieur à 30°C et de préférence inférieur à 120°C. Le point de fusion de la cire peut être mesuré à l'aide d'un calorimètre à balayage différentiel (DSC), par exemple celui proposé par la société METTLER sous la référence DSC 30.

Les cires peuvent être hydrocarbonées, siliconées ou fluorées et être d'origine naturelle minérale, animale, végétale ou d'origine synthétique. Elles peuvent être polaires ou apolaires.

Comme cires apolaires on peut citer les cires microcristallines, les cires de paraffines, l'ozokérite, les cires de polyéthylènes.

Les cires polaires sont de préférence hydrocarbonées et encore plus préférentiellement choisies parmi les cires esters comprenant au moins une fonction ester et les cires alcools comprenant au moins une fonction alcool.

Les cires esters sont de préférence choisies parmi les esters d'acide(s) gras et/ou d'alcools gras sous forme solide à température ambiante et à pression atmosphérique. Ces cires esters peuvent être issues d'huiles animales ou végétales hydrogénées.

Les cires alcools ont de préférence choisies parmi les alcools gras sous forme solide à température ambiante et à pression atmosphérique tels que l'alcool cétylique ou l'alcool cétylstéarylique.

Les cires d'origine naturelle sont de préférence choisies parmi la cire d'abeille, la cire de carnauba, la cire de candellila, la cire de son de riz, la cire d'ouricury, la cire d'alfa, la cire de fibres de liège, la cire de canne à sucre, la cire du Japon, la cire de sumac, la cire de montan, la cire d'orange, la cire de laurier, la cire de pomme, le beurre de karité. De préférence la ou les cires est ou sont choisies parmi les cires végétales. Mieux les compositions de l'invention ne contiennent que des cires végétales. Plus préférentiellement la ou les cires est ou sont choisies parmi la cire de carnauba, la cire de candellila et le beurre de karité.

De préférence les compositions selon l'invention contiennent au moins une huile végétale et/ou au moins une cire végétale.

Dans les compositions selon l'invention la teneur en corps gras peut varier de 1% à 98% en poids, mieux de 10% à 95% en poids, encore mieux de 20% à 95% en poids par rapport au poids total de la composition.

Les compositions selon l'invention peuvent comprendre un ou plusieurs agents tensioactifs choisis parmi les agents tensioactifs anioniques, non-ioniques, cationiques ou amphotères.

Les compositions selon l'invention peuvent en outre comprendre un ou plusieurs additifs classiques bien connus dans la technique.

A titre d'exemples d'additifs utilisables selon l'invention, on peut citer les polymères associatifs ou non associatifs, ioniques ou non-ioniques et en particulier les polymères épaississants, les épaississants minéraux, les filtres UVA et/ou UVB, les agents émollients, les solvants, les vitamines, les pro-vitamines, les acides aminés, les parfums ou arômes, les agents peptisants, les agents conservateurs, les agents antioxydants, les agents alcalinisants ou acidifiants (notamment pour réguler le pH des compositions aqueuses ou pour salifier les ingrédients), les agents fixateurs de couleur, les agents matifiants comme le talc, le kaolin, la silice ou le dioxyde de titane. Les compositions cosmétiques peuvent en plus des ingrédients ci dessus contenir des agents spécifiques à ce domaine comme des agents antirides, des agents hydratants des matières kératiniques, des agents adoucissants pour la peau, des agents kératolytiques, des agents anti-sébum. Elles peuvent être aqueuses ou anhydres. Dans une variante préférée de l'invention les compositions cosmétiques sont anhydres.

Les domaines d'utilisation des matières premières colorantes selon l'invention peuvent être très variés. Il peut s'agir par exemple d'une mise en œuvre dans des produits alimentaires ( boissons, produits laitiers, glaces, desserts divers..), dans des produits ménagers ( lessives solides ou liquides, nettoyants ménagers, désodorisants, produits anti-insectes...), dans des matériaux industriels ( plastiques, peintures, vernis, lasures...), dans des produits pour impression (encres), dans l'industie textile notamment pour les opérations de teinture, dans la parapharmacie et dans les produits cosmétiques.

Encore plus préférentiellement, les matières premières colorantes selon l'invention sont utilisées pour modifier la couleur des substrats sur lesquels on les dépose.

Préférentiellement les matières colorantes selon l'invention sont utilisées dans le domaine cosmétique, encore mieux dans des compositions de maquillage.

Par composition pour le maquillage, on entend au sens de la présente invention une composition apportant de la couleur au substrat anatomique sur lequel elle est appliquée.

Ces substrats anatomiques peuvent être les cheveux, la cavité buccodentaire, la peau, les lèvres, les ongles, les cils, les sourcils. De préférence les substrats cosmétiques sur lesquels sont appliquées les matières premières colorantes de l'invention sont la peau, les lèvres, les ongles et les cils. Les matières premières colorantes de l'invention sont alors préférentiellement utilisées dans des compositions pour le maquillage de la peau ou des lèvres ou des ongles ou des cils.

Pour optimiser la couvrance des substrats par les matières premières colorantes de l'invention on peut incorporer dans les compositions de l'invention un ou plusieurs composés solides lamellaires insolubles dans l'eau et non siliciés qui peuvent être choisis parmi la guanine, l'oxyde de zinc et les stéarates de métaux divalents ( notamment stéarate de zinc ou de magnésium) ou leurs mélanges.

Par composé insoluble dans l'eau, on entend au sens de la présente invention un composé dont la solubilité dans l'eau est inférieure à 0,25% (0,25g pour 100ml), de préférence inférieure à 0,05%, mieux inférieure à 0,005% à température ambiante (de préférence 25°C) et à pression atmosphérique (1,013×10⁵ Pa).

Par composé lamellaire on entend au sens de la présente invention que le composé à température ambiante et à pression atmosphérique présente un arrangement des cristaux en feuillets ou plaquettes.

Par composé non silicié on entend au sens de la présente invention un composé ne comprenant pas d'atome de silicium dans sa formule chimique.

Par composé solide on entend qu'à température ambiante et à pression atmosphérique le composé lamellaire insoluble dans l'eau se présente se présente sous forme d'un solide, cristallisé ou amorphe.

Les compositions de maquillage citées peuvent notamment, de manière non limitative, se présenter sous forme de rouges à lèvres, de crayons pour les lèvres, de fluides à lèvres, de mascaras, de fonds de teint, de fards à joues, d'ombres à paupières, d'eye liners.

L'invention couvre également un procédé de maquillage de la peau ou des lèvres ou des cils ou des ongles, de préférence de la peau ou des lèvres , et encore plus préférentiellement des lèvres consistant à appliquer sur le substrat anatomique (peau ou lèvres ou cils ou ongles) une composition cosmétique comprenant une ou plusieurs matières colorantes selon l'invention telles que décrites ci dessus, sans rinçage subséquent.

### [Exemples]

Les exemples ci-dessous illustrent l'invention sans toutefois en limiter la portée.

### Exemple 1

Des tomates rouges ( Solanum Lycopersicum) sont hachées, déshydratées et broyées finement. Le produit obtenu est soumis à une extraction par le dioxyde de carbone supercritique à une pression de 60 MPa et à une température de 80°C pendant 8 Heures avec un flux de dioxyde de carbone de 35 Kg de dioxyde de carbone par heure. L'extrait obtenu est ensuite microencapsulé avec de la maltodextrine préalablement dissoute dans l'eau chaude, conservée une nuit à 4°C pour réhydratation. Pour cela on mélange l'extrait avec la solution de maltodextrine pour atteindre une concentration finale en solide de 20% en poids et le mélange obtenu est atomisé avec un appareil adéquat pour fournir en final la matière première colorante de l'invention A.

Cette matière colorante présente à la fois une absence d' odeur et de couleur parasites avec une bonne conservation dans le temps et face à la lumière du pouvoir colorant, une facilité de mise en œuvre dans tous les milieux et une bonne innocuité. Un tel résultat ne peut être obtenu avec des microcapsules de chitosane ou d'alginate.

On peut utiliser cette matière colorante dans la composition suivante selon le tableau 1. Cette composition se présente sous la forme d'un baton de rouge à lèvres.

**[Tableaux1]**

| Ingrédient | Quantité en g pour 100g de composition |
|---|---|
| Matière première colorante issue de tomates rouges(A) | 6 |
| Oxyde de zinc | 14,4 |
| Stéarate de zinc | 1,6 |
| Huile de ricin | 19,25 |
| Huile de tournesol | 32,27 |
| Cire de Carnauba | 18 |
| Beurre de karité | 5,6 |
| Alun de potassium | 1,83 |
| Carbonate de sodium | 0,87 |
| Tocopherol | 0,18 |

### Exemple 2

Cet exemple ne diffère de l'exemple 1 que par l'étape d'enrobage qui est réalisée cette fois avec une association protéine de blé soluble (SWP 100) en présence de gomme arabique dans un rapport massique ½ et à pH 3 , ceci via une technique de coacervation. On obtient une matière première colorante B.

Les résultats sur les caractéristiques de la matière colorante B sont similaires à ceux indiqués pour la matière colorante A de l'exemple 1.

On peut alors réaliser un rouge à lèvres dont la composition est celle obtenue en remplaçant dans le tableau 1 la matière première colorante A par une quantité pondérale égale de matière première colorante B.

### Exemple 3

Des racines de garance (Rubia Tinctorum) sont déshydratées sous vide puis broyées finement. Le produit obtenu est soumis à une extraction en présence d'éthanol pur à pression atmosphérique et à une température de 45°C en présence d'ultrasons (25 KHz) avec une puissance de 100W pendant 90 minutes. Après filtration le solvant est évaporé et l'extrait obtenu est microencapsulé avec de la maltodextrine comme dans l'exemple 1. On obtient une matière première colorante C.

Les résultats sur les caractéristiques de la matière colorante C sont similaires à ceux indiqués pour la matière colorante A de l'exemple 1.

On peut alors réaliser un rouge à lèvres dont la composition est celle obtenue en remplaçant dans le tableau 1 la matière première colorante A par une quantité pondérale égale de matière première colorante C.

### Exemple 4

On traite de la pulpe de tomate ( Solanum Lycopersicum) par un mélange enzymatique (pectinase+cellulase+protéase) à 45°C pendant 1heure et après concentration, on fait agir de l'acétate d'étyle à pression atmosphérique dans un rapport massique concentré/solvant de 1:5 et à une température de 45°C pendant 2heures. On peut aussi ajouter un traitement ultrasonique avec une fréquence de 30 KHz pendant 30 mn avec une puissance de 80W. Après filtration le solvant est évaporé et l'extrait obtenu est microencapsulé avec de la maltodextrine comme dans l'exemple 1. On obtient une matière première colorante D1 (sans traitement ultrasonique) ou D2 (avec traitement ultrasonique).

Les résultats sur les caractéristiques des matières colorantes D1 et D2 sont similaires à ceux indiqués pour la matière colorante A de l'exemple 1.

On peut alors réaliser un rouge à lèvres dont la composition est celle obtenue en remplaçant dans le tableau 1 la matière première colorante A par une quantité pondérale égale de matière première colorante D1 ou D2.

### Exemple 5

De l'écorce de chataignier (Castanea sativa) est traitée par le procédé d'extraction décrit dans l'exemple 1 utilisant le dioxyde de carbone supercritique. L'extrait obtenu est ensuite microencapsulé par de la maltodextrine comme dans l'exemple 1.

On obtient une matière première colorante E.

Les résultats sur les caractéristiques de la matière colorante E sont similaires à ceux indiqués pour la matière colorante A de l'exemple 1.

## Revendications

1. Matière première colorante constituée d'un extrait colorant issu de la totalité ou d'une ou plusieurs parties d'une ou plusieurs plantes à partir d'un procédé comprenant les étapes suivantes: (a) une phase d'extraction comprenant au moins une extraction (aᵢ) avec action d'un ou plusieurs agents exogènes choisis parmi les solvants sous pression, les enzymes, les ondes radioélectriques de fréquences allant de 16 Khz à 300 GHz (b) un enrobage de l'extrait issu de la phase d'extraction (a) avec un ou plusieurs agents issus d'une ou plusieurs plantes.

2. Matière première colorante selon la revendication 1 pour laquelle l'extraction (aᵢ) est réalisée avec action d'un ou plusieurs solvants exogènes qui sont liquides à température ambiante (25°C) et à pression atmosphérique (1,013×10⁵ Pa) et qu'on utilise sous pression, choisis de préférence parmi les composés ayant de 0 à 12 atomes de carbone, plus préférentiellement choisis parmi l'hexane, l'acétate d'éthyle l'acétone, l'eau, l'éthanol et leurs mélanges et encore plus préférentiellement parmi l'éthanol et les mélanges éthanol eau .

3. Matière première colorante selon la revendication 1 pour laquelle l'extraction (aᵢ) est réalisée avec action d'un ou plusieurs composés exogènes qui sont gazeux à température ambiante (25°C) et à pression atmosphérique (1,013×10⁵ Pa) et qu'on utilise sous pression sous forme liquide ou pseudo liquide, choisis de préférence parmi les composés ayant de 0 à 12 atomes de carbone et plus préférentiellement parmi le butane, le 1,1,1,2 tétrafluoroéthane, le 1,1 difluoroéthane, le 1,3,3,3 tétrafluoro prop-1 ène, le dioxyde de carbone supercritique.

4. Matière première colorante selon les revendications 1 et 3 pour laquelle l'extraction (aᵢ) est réalisée avec le dioxyde de carbone supercritique.

5. Matière première colorante selon l'une quelconque des revendications 1 à 4 pour laquelle le solvant lors de l'extraction (aᵢ) est soumis à une pression allant de 1 à 200 MPa, mieux de 3 à 150 MPa, encore mieux de 10 à 100 MPa.

6. Matière première colorante selon l'une quelconque des revendications 1 à 5 pour laquelle l'extraction (aᵢ) est réalisée avec une ou plusieurs enzymes exogènes choisies parmi les hydrolases et les lyases.

7. Matière première colorante selon l'une quelconque des revendications 1 à 6 pour laquelle l'extraction (aᵢ) est réalisée avec une ou plusieurs enzymes exogènes choisies parmi les décarboxylases, les aldolases, les déshydratases, les estérases, les peptidases, les glycosidases et les phosphatases, et encore plus préférentiellement parmi les cellulases, les beta glucosidases, les hémicellulases, les xylanases, les glucanases, les béta glucanases, les pectinases, les amylases, les alpha amylases, les phospholipases, les beta-mannanases, les galactanases , les arabinanases, les laminarinases, les galacturonases, les polygalacturonases, les phytases, les exocellulases, les phénylestérases et les protéases.

8. Matière première colorante selon l'une quelconque des revendications 1 à 7 pour laquelle l'extraction (aᵢ) est réalisée à température peu élevée sans chauffage de 4°C à 40°C, mieux de 20°C à 40°C ou avec un chauffage allant de 41°C à 200°C, mieux de 45°C à 160°C, encore mieux de 45°C à 100°C.

9. Matière première colorante selon l'une quelconque des revendications 1 à 8 dans laquelle le ou les agents d'enrobage sont des polysaccharides d'origine végétale choisis parmi les dextrines dont les maltodextrines et cyclodextrines, les dextranes, les celluloses, les amidons, les pectines et leurs mélanges.

10. Matière première colorante selon l'une quelconque des revendications 1 à 8 pour laquelle le ou les agents d'enrobage sont des polypeptides, mieux des protéines issues de plantes choisies parmi les céréales, les légumineuses, les oléagineuses et de préférence parmi le blé, le maïs, l'orge, l'avoine , le soja, le coton, le riz, le pois chiche, le petit pois, le seigle, le froment, le millet, la fève, le lupin, la lentille, l'épautre, le sésame, le sarrazin, le fénugrec, le tournesol, le lin.

11. Matière première colorante selon l'une quelconque des revendications 1 à 10 dans laquelle l'extrait colorant est issu d'une ou plusieurs des plantes suivantes : garance, tomate, quebracho, pernambouc, châtaignier, curcuma, sorgho, cachou, rocouyer, sappan, genipa, indigotier , chou rouge, réséda, radis rouge, sumac ou mieux est issu des plantes suivantes: garance, tomate, sorgho, châtaignier, radis rouge, sumac.

12. Composition comprenant au moins une matière première colorante selon l'une quelconque des revendications 1 à 11 , de préférence dans une concentration allant de 0,001% à 60% en poids, plus préférentiellement de 0,1% à 50% en poids, mieux de 0,5% à 40% en poids, encore mieux de 1% à 35% en poids par rapport au poids total de la composition.

13. Composition selon la revendication 12 comprenant au moins une huile végétale et/ou au moins une cire végétale.

14. Composition selon l'une queconque des revendications 12 ou 13 destinée au maquillage de la peau, des lèvres, des cils et des sourcils et se présentant de préférence sous la forme d'un rouges à lèvres, d'un crayon pour les lèvres, d'un fluide à lèvres, dun mascara, d'un fond de teint, d'un fard à joues, d'une ombre à paupières, d'un eye liner.

15. Procédé de maquillage de la peau ou des lèvres ou des cils ou des ongles, de préférence de la peau ou des lèvres, et encore plus préférentiellement des lèvres consistant à appliquer sur le substrat anatomique une composition selon l'une quelconque des revendications 12 à 14 sans rinçage subséquent.
